# EUROPEAN PATENT APPLICATION

(11) **EP 4 610 207 A1**
(43) Date of publication of application: **03.09.2025**
(21) Application number: 23881678.9
(22) Date of filing: 16.10.2023
(51) Int. Cl.: B65H 23/195, A61M 15/00

(54) **WINDING DEVICE FOR MATERIAL STRIP, AND MEDICAMENT DISPENSER**

(30) Priority: 27.10.2022 CN 202211339731
(71) Applicant: Transpire Bio Inc, Plantation, FL 33324 (US)
(72) Inventor: LIU, Yongwei, Shenzhen, Guangdong 518102 (CN); ZHANG, Xieen, Shenzhen, Guangdong 518102 (CN); OUYANG, Guanglin, Shenzhen, Guangdong 518102 (CN)
(74) Representative: Latscha Schöllhorn Partner AG
(86) International application number: PCT/CN2023/124818
(87) International publication number: WO 2024/088099

(57) **Abstract**

Disclosed is a winding device for a cover strip. The winding device includes: a driver, including a driving shaft around which a first shoulder portion having a first inclined surface is disposed, and configured to be rotatable in a rotating direction; a guide, installed on the driving shaft, and having a second inclined surface matched with the first inclined surface of the first shoulder portion; a take-up reel, connected with guide in a relatively nonrotatable manner; and an elastic body, received between guide and the take-up reel for applying a preload to guide, where the winding device is constructed such that the take-up reel is rotatable in the rotating direction under the driving of drive, when the force acting on the take-up reel via the cover strip is greater than a preset value, the take-up reel is also rotatable relative to drive. A medicine dispenser including the winding device for a cover strip is further disclosed.

## Description

### CROSS-REFERENCE TO RELATED APPLICATIONS

This application claims priority to Chinese Patent Application 202211339731. X, filed on October 27, 2022, which is incorporated by reference in its entirety.

### TECHNICAL FIELD

The disclosure generally relates to dry powder inhalers, and, more specifically, to a winding device for a blister strips used in dry powder inhalers.

### BACKGROUND

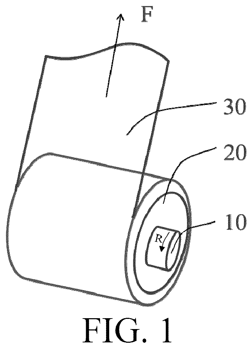

In conventional medicine dispensers, such as dry powder inhalers, winding of a blister strip is an integral part of the device . As shown in FIG. 1, a conventional winding device for a cover strip (commonly a blister strip) generally includes a winding shaft 10 and a take-up reel 20 installed on the winding shaft 10. The winding shaft 10 can be connected with a power source through a transmission mechanism, and can thus rotate under the driving of the power source (for example, a manual power source or a motor). The winding shaft 10 drives the take-up reel 20 to rotate, so that a cover strip 30 is wound onto the take-up reel 20.

The take-up reel 20 generally rotates in the rotating direction R at a fixed rotating speed that is the same as or at a particular transmission ratio as a feeding mechanism (not shown), which correspondingly winds up the cover strip 30 provided by the feeding mechanism. One of the problems with this design is that as the number of layers of the cover strip 30 wound by the take-up reel 20 increases, a winding diameter of an outermost circle of the take-up reel 20 (i.e., the effective winding diameter) also increases. As a result, the winding linear speed of the take-up reel 20 is higher than a feeding speed of the feeding mechanism, and a tension F received by the cover strip 30 between the take-up reel 20 and the feeding mechanism increases. When the tension F is greater than a preset value, the cover strip 30 can stretched thin due to excessive tension, so that thickness unevenness or even tearing of the cover strip 30 can occur.

For a medicine dispenser like a dry powder inhaler, the length increase of the cover strip wound with each rotation of the take-up reel can cause the change of relative positions of other components in the medicine dispenser. For example, for a medicine dispenser using a blister medicine strip, in normal operation, through the medicine dispensing action of the medicine feeding mechanism, a blister opening in the blister medicine strip after stripping is directly aligned with an airway structure of an intake manifold, so that a user can conveniently intake the medicine. Meanwhile, the take-up reel correspondingly winds the stripped cover strip, for example, the cover strip of a medicine strip cover plate, so as to avoid the interference with the medicine obtaining process by the user.

However, when the effective winding diameter of the take-up reel increases, the length of the cover strip wound as each rotation of the take-up reel increases. At this moment, the action force received by the blister opening from the wound cover strip correspondingly increases, so that the blister opening moves under the action of the wound cover strip, resulting in large change or even complete misalignment of the relative position with the airway structure of the medicine dispenser. As a result of this misalignment, the user cannot obtain the medicine or the sufficient medicine, and the medicine dispenser cannot be normally used.

### SUMMARY

In order to alleviate or eliminate the above-described problems, in an aspect described herein, a winding device for a cover strip is provided, the winding device comprising:
a driver, including a driving shaft around which a first shoulder portion having a first inclined surface is disposed, and configured to be rotatable in a rotating direction;
a guide, installed on the driving shaft, and having a second inclined surfaces matched with the first inclined surface of the first shoulder portion;
a take-up reel, connected with guide in a relatively nonrotatable manner; and
an elastic body, received between guide and the take-up reel for applying a preload to guide;
where the winding device is constructed such that the take-up reel is rotatable in the rotating direction under the driving of drive, when the force acting on the take-up reel via the cover strip is greater than a preset value, the take-up reel is also rotatable relative to drive.

According to an implementation of the disclosure, an included angle between a normal vector of the first inclined surface and the rotating direction of drive is an acute angle.

According to an implementation of the disclosure, one first inclined surface is provided, and the first inclined surface is distributed over the entire circumference of the first shoulder portion around the driving shaft.

According to an implementation of the disclosure, a plurality of first inclined surfaces are provided, and each of the first inclined surfaces is uniformly distributed in the peripheral direction over the entire circumference of the first shoulder portion around the driving shaft.

According to an implementation of the disclosure, two first inclined surfaces are provided. According to an implementation of the disclosure, the elastic body is a compressed spring.

According to another implementation of the disclosure, guide is provided with an receiving space disposed around the driving shaft and configured to accommodate the compressed spring.

According to another implementation of the disclosure, the receiving space is constructed into a first tubular structure with a bottom, and a through hole for inserting the driving shaft is formed in the bottom.

According to another implementation of the disclosure, an axially penetrating notch is formed on the outer periphery of the receiving space.

According to another implementation of the disclosure, the take-up reel is constructed into a second tubular structure with a top, a through hole is formed in the top, a protruding portion is formed in the inner surface of the take-up reel, and the take-up reel is fitted over the radial outer side of guide through the engagement of the protruding portion and the notch.

According to another implementation of the disclosure, a protruding rib portion is disposed at the upper end of the driving shaft, and the top of the take-up reel is abutted against the bottom surface of the protruding rib portion.

According to another implementation of the disclosure, the take-up reel is axially and eccentrically provided with a cut-out portion, and a cover strip fin is vertically disposed on the outermost circumference of the cut-out portion.

According to another implementation of the disclosure, drive includes a driving base plate, and the driving shaft and the driving base plate are integrally formed or separately formed.

According to another implementation of the disclosure, in a case that the driving shaft and the driving base plate are separately formed, the driving shaft is detachably installed on the driving base plate.

According to another implementation of the disclosure, the driving base plate is constructed as a gear.

According to another implementation of the disclosure, a second shoulder portion is formed at the bottom of guide, and the second inclined surface is formed on the second shoulder portion.

According to another implementation of the disclosure, the position and the quantity of the second shoulder portion are in one-to-one correspondence to those of the first inclined surface.

According to another implementation of the disclosure, a first shoulder stop portion and a second shoulder stop portion that are mutually clamped are respectively formed on the first shoulder portion and the second shoulder portion.

For the winding device according to the first aspect of the disclosure, based on the cover strip tension, the take-up reel can rotate relative to drive when the effective winding diameter thereof increases, so that the motion compensation for the take-up reel winding the cover strip is realized, the length of the take-up reel winding the cover strip as each rotation keeps consistent, and thus the condition of cover strip fracture due to excessive cover strip tension is avoided.

According to the second aspect of the disclosure, a medicine dispenser is provided, and the medicine dispenser includes the winding device for a cover strip according to any of the implementations in the first aspect of the disclosure.

On the basis of conforming to the common knowledge in the art, the above implementations can be freely combined to obtain preferred implementations of the disclosure.

### BRIEF DESCRIPTION OF THE DRAWINGS

The invention will now be described by way of reference to the following drawings, of which:
FIG. 1 is a schematic diagram of a winding device for a cover strip in the prior art;
FIG. 2 is a schematic exploded view of a winding device for a cover strip according to an embodiment;
FIG. 3 is a schematic diagram of a driver in the winding device in FIG. 2;
FIG. 4 is a schematic diagram of a guide in the winding device in FIG. 2;
FIG. 5 is a schematic diagram of a take-up reel in the winding device in FIG. 2;
FIG. 6 is a schematic diagram of the take-up reel in FIG. 5 in another view angle;
FIG. 7 is a cross-sectional view of the winding device in FIG. 2 in an assembled state;
FIG. 8 is a cross-sectional view of a part of the winding device in FIG. 2 in a synchronous working state;
FIG. 9 is a partial cross-sectional view of the winding device in FIG. 2 in a motion compensation working state;
FIG. 10 is an exploded view of another variation of drive in the winding device in FIG. 2;
FIG. 11 is a bottom view of a driving base plate of drive in FIG. 10;
FIG. 12a to FIG. 12b show cross-sectional views of the driving base plate of drive in FIG. 11 from different angles respectively;
FIG. 13 is a cross-sectional view of drive in FIG. 10 after assembly;
FIG. 14 is a medicine dispenser according to an embodiment;
FIG. 15 is a schematic diagram of a medicine dispensing device and a medicine strip in the medicine dispenser in FIG. 14; and
FIG. 16 is a schematic exploded view of a gear transmission system in the medicine dispensing device in FIG. 15.

### DETAILED DESCRIPTION

In order to describe the objectives, the technical solution and the advantages of the disclosure more clearly, the technical solution of embodiments of the disclosure will be clearly and completely described hereinafter with reference to accompanying drawings according to a plurality of implementations of the disclosure. It should be understood that described embodiments are only a part of embodiments of the disclosure, rather than all embodiments. All other embodiments obtained by a person of ordinary skill in the art based on the embodiments of the disclosure without making creative efforts shall fall within the protection scope of the disclosure.

In the specification, claims, and brief description of the drawings of the disclosure, the terms "include", "have", "contain", and the like are open-ended terms. Therefore, a device "including" or "having", for example, one or more elements, refers to the device having one or more elements, but is not limited to having only the one or more elements.

The terms "first", "second", and the like in the specification, claims and brief description of the drawings of the disclosure are used for distinguishing different objects, and are not used for describing a particular sequence or primary and secondary relationship. In addition, terms "first" and "second" are used merely for the purpose of description, and shall not be construed as indicating or implying relative importance or implying a quantity of indicated technical features. Therefore, a feature limited by "first" or "second" can explicitly or implicitly include one or more of the features.

In addition, orientation or positional relationships indicated by terms "top", "bottom", "axial direction", "radial direction", "peripheral direction", "outer periphery", "outer side", and the like indicating the orientation in the descriptions of the disclosure are based on the orientation or the positional relationship shown in the drawings, are only used for convenience of describing the disclosure and description simplification, are not intended to indicate or imply that the referenced device or element must have a particular orientation or be constructed and operated in a particular orientation, and therefore cannot be understood as a limitation to the disclosure.

In the description of the disclosure, it should be noted that unless otherwise explicitly specified or defined, the terms "dispose", "be provided with", "have", and "install" should be understood in a broad sense. For example, "install" can be "fixed connection", or "detachable connection", or "integral connection", can be direct connection, can be indirect connection through an intermediate medium, or can be internal communication between two components. For those of ordinary skill in the art, the specific meaning of the above-mentioned terms in the disclosure can be understood according to specific circumstances.

The winding device described herein can be used in a medicine dispenser. The medicine dispenser is a device that dispenses medicaments for administering a patient. In an embodiment, the medicine dispenser is a dry powder inhaler ("DPI") where medicine is delivered into the lungs and/or nasal cavities through inhalation.

A medicine dispenser described herein generally comprises a housing, a medicine carrier strip for carrying powdered medicine (such as a blister strip), an administration device for dispensing medicine, a stripping device for separating a cover strip from the medicine carrier strip, and a winding device for winding the medicine carrier strip in cooperation with the administration device and the stripping device.

A conventional medicine carrier strip often includes a plurality of blisters sealed with a cover strip over the top thereof, each blister holding a powdered medicine at a discrete dose. The medicine carrier strip is disposed in the housing. The administration device moves the blister strips in the housing to dispense the medicine. The stripping device strips a cover strip from the blister strip, so that a certain dose of powdered medicine is obtained and dispensed to an airway of an intake manifold. A user can conveniently inhale the powdered medicine through a suction nozzle of a medicine powder suction device and the airway of the intake manifold for treatment. At the same time, a winding device can wind the stripped cover strip to avoid interference of the stripped cover strip with other components of the medicine dispenser.

In practice, the winding device moves synchronously with the administration device and cooperatively winds the cover strip by rotating a specified degree in cooperation with the administration device. However, in a process that the winding device continuously winds the cover strip, the effective winding diameter of a take-up reel will gradually increase. Therefore, the linear speed of the take-up reel is increasingly faster than the administration speed of the administration device as more cover strip is wound on the winding device, resulting in increasing tension on the cover strip between the winding device and the administration device. Under the action of tensile force of the wound cover strip, the relative positions of the medicine on a blister strip will change, and misalignment of the blisters and airway can occur. Additionally, if the tension becomes too high, cover strip fractures and tears can also occur.

In order to address the increasing effective winding diameter of the cover strip with each rotation of the winding device and prevent misalignment of the blister opening after stripping with the airway of the intake manifold, i.e., misalignment of the blister and airway, and reduce the chances of cover strip fractions and tears caused by excessive tension received by the cover strip, an improved winding device is needed. Specifically, a winding device is needed that compensates for the increasing length of the wound cover strip from each rotation due to the increase of the effective winding diameter of the take-up reel. In this way, the length consistency of the wound cover strip as each rotation of the take-up reel is ensured, ensuring that the relative position change between a medicine-containing blister, airway, and stripped cover strip is maintained and excessive tension is avoided. Consequently, such a winding device would prevent or reduce the probability of a medicine dispenser failing to operate properly. The winding device according to a first aspect of the disclosure will be described in detail hereafter in combination with the accompanying drawings.

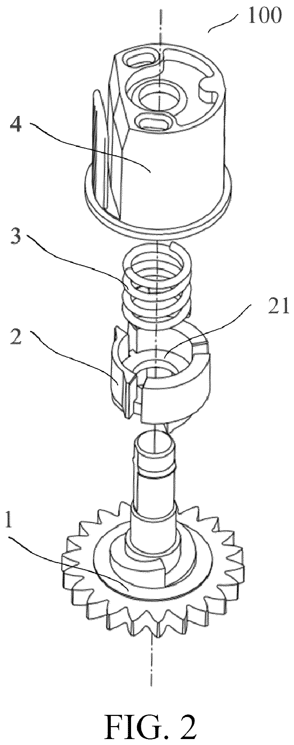

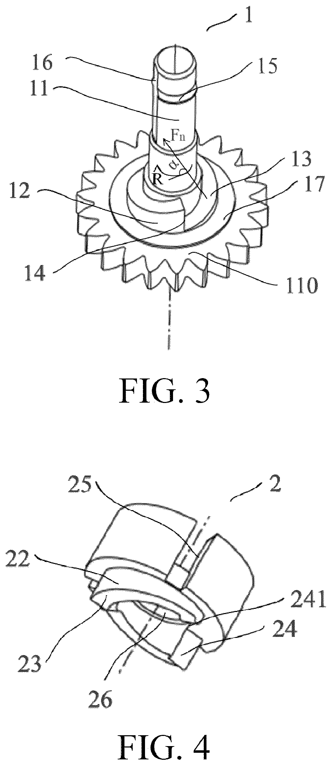

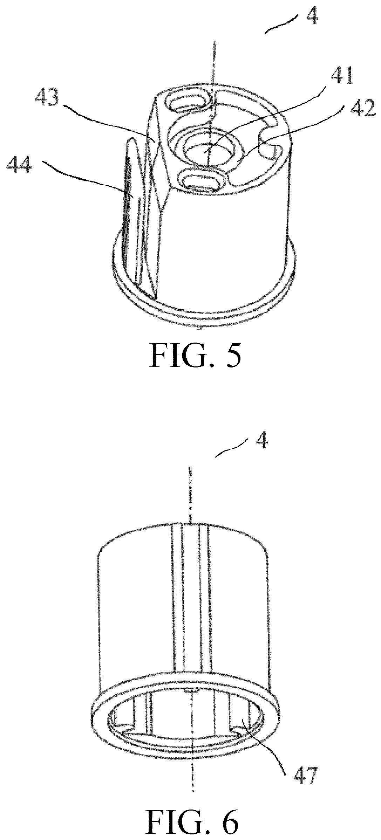

FIG. 2 is a winding device 100 for a cover strip according to the disclosure. Referring to FIG. 2, the winding device 100 includes a driver 1, a guide 2, an elastic body 3, and a take-up reel 4. FIG. 3 illustratively shows a structure of driver 1. FIG. 4 illustratively shows a structure of guide 2. FIG. 5 and FIG. 6 illustratively show a structure of the take-up reel 4. Referring to FIG. 2 to FIG. 6, drive 1 includes a driving shaft 11. A first shoulder portion 12 having a first inclined surface 13 is disposed on the outer periphery of the driving shaft 11. The first inclined surface 13, for example, can be constructed into a spiral shape surrounding the driving shaft 11. Driver 1 further includes a driving base plate 110 positioned at the bottom thereof, and is configured to cooperate with an external power source through the driving base plate 110, to rotate under the driving of the external power source.

Guide 2 is installed on the driving shaft 11 and has a second inclined surface 23 matched with the first inclined surface 13 of the first shoulder portion 12. In an initial installation state, the first inclined surface 13 of the first shoulder portion 12 and the second inclined surface 23 of guide 2 can be abutted against each other, so as to form shape fit connection. In other words, the first inclined surface 13 and the second inclined surface 23 can be constructed to present a spiral shape surrounding the driving shaft 11, and the two have the same helix angle. Guide 2 is further constructed to be rotatable along with drive 1, or movable relative to drive 1 along the first inclined surface 13 in a direction opposite to the rotating direction R of drive 1.

The take-up reel 4 is fitted over guide 2, and is connected to guide 2 in a relatively nonrotatable manner. The connection in a relatively nonrotatable manner refers to connection in which relative rotation is prevented in the circumferential direction of drive 1 (i.e. synchronous rotation), while relative movement is allowed in the axial direction. Therefore, under the driving of guide 2, the take-up reel 4 is rotatable together with guide 2 along with drive 1 under the driving of drive 1, or is rotatable relatively to drive 1 in a direction opposite to the rotating direction R of drive 1 to realize motion compensation.

The elastic body 3 is disposed between guide 2 and the take-up reel 4 so as to apply a preload to guide 2. Therefore, the second inclined surface 23 presses against the first inclined surface 13. When the preload applied by the elastic body 3 to guide 2 changes, the friction force between the second inclined surface 23 of guide 2 and the first inclined surface 13 of drive 1 also correspondingly changes.

Preferably, guide 2 is provided with a receiving space 21 disposed around the driving shaft 11, so that the elastic body 3 can be better positioned between guide 2 and the take-up reel 4. Specifically, in some embodiments, the elastic body 3 is a compressed spring, and sleeves the driving shaft 11. In some instances, the elastic body 3 can be any other compressive material known to the skilled artisan.

Referring to FIG. 3, drive 1 includes a driving base plate 110 positioned at the bottom thereof, a driving shaft 11 for installing and positioning other components, a first shoulder portion 12 for guiding and cooperating with guide 2, and a step portion 17 for supporting the take-up reel 4. The driving shaft 11 is integrally formed with the driving base plate 110 in some embodiments, is vertically disposed on the driving base plate 110, and axially extends in a direction away from the driving base plate 110. A protruding rib portion 15 radially protruding outwards from the driving shaft 11 is disposed around the outer periphery of the driving shaft 11 at the top end of the driving shaft 11 distal to the driving base plate 110. From a top to a bottom of the protruding rib portion 15, the protruding rib portion 15 is provided with an inclined surface axially and outwards inclined in the radial direction so as to guide and install the take-up reel 4 to the bottom surface of the protruding rib portion 15. At the same time, the maximum peripheral diameter of the protruding rib portion 15 is greater than a through hole 41 of the take-up reel 4, so that the top of the take-up reel 4 after the installation abuts against the bottom surface of the protruding rib portion 15. Therefore, the take-up reel is unable to axially move in a direction away from the bottom of drive 1.

In some embodiments, the end of the driving shaft 11 distal to the driving base plate 110 can further be provided with a planar side-cut portion 16 parallel to the axial direction, and the planar side-cut portion 16 passes through the protruding rib portion 15 in the axial direction so as to increase an installation space for the take-up reel 4 and the driving shaft 11. Therefore, it is convenient to install the take-up reel 4 at the bottom side of the protruding rib portion 15.

In some embodiments, the driving base plate 110 of drive 1 is constructed as a gear. Drive 1 includes a gear positioned at the bottom and driving shaft 11 disposed in the center of the gear. Drive 1 is engageable with external gears and racks through teeth of the gear, so as to rotate under the driving of a power source. Alternatively, the driving base plate 110 is constructed as a pulley. In some cases, the pulley cooperates with an external transmission component and power is sourced through a belt disposed on the pulley, where the pully rotates under movement from the power source.

An annular step portion 17 formed around the driving shaft 11 is further disposed on the driving base plate 110, and the step portion 17 protrudes from the top surface of the driving base plate 110. In a preliminary installation state of the winding device 100, the step portion 17 is in contact with the bottom wall of the take-up reel 4 to position and support the take-up reel 4, and can cooperate with the protruding rib portion 15 to limit the axial motion of the take-up reel 4. In some instances, an outer diameter of step portion 17 is smaller than an outer diameter of the take-up reel 4.

The driving base plate 110 of drive 1 is further provided with a first shoulder portion 12, and a first inclined surface 13 is formed at the top surface of the first shoulder portion 12. Referring to FIG. 3, an included angle α between a normal vector Fₙ of the first inclined surface 13 and the rotating direction R of drive 1 is an acute angle. That is, in the rotating direction R, the distance from the first inclined surface to the bottom surface of the driving base plate 110 gradually decreases. By setting the included angle α between the normal vector Fₙ of the first inclined surface 13 and the rotating direction of drive 1 to be an acute angle, under a certain condition, guide 2 is movable relative to drive 1 along the first inclined surface 13 in a direction opposite to the rotating direction R.

The first shoulder portion 12 can be provided with only one first inclined surface 13. In this case, the first inclined surface 13 is formed on the entire circumference of the first shoulder portion 12 around the driving shaft 11. When only one first inclined surface 13 is provided, since the circumference length of the first inclined surface 13 is long enough, in a rotation compensation process of the winding device 100, the length required for guide 2 to move relative to drive 1 along the first inclined surface 13 can be met. At the same time, since the circumference length of the first inclined surface 13 is long enough, it can be ensured that the motion of guide 2 along the first inclined surface 13 is continuous. In this way, the second inclined surface 23 of guide 2 will not completely separate from the first inclined surface 13, the bouncing of guide 2 in the motion process is avoided, and the vibration and noise of the winding device 100 in the cover strip winding process are further avoided.

The first shoulder portion 12 can also be provided with a plurality of first inclined surfaces 13, and the specific quantity can be selected and determined according to practical requirements. When a plurality of first inclined surfaces 13 are provided, the inclined surfaces are uniformly distributed over the entire circumference of the first shoulder portion 12 around the driving shaft 11, and the top surface of the first shoulder portion 12 is in a sawtooth shape in the peripheral direction. The plurality of first inclined surfaces 13 can be uniformly distributed successively on the circumference formed by the first shoulder portion 12, and can also be uniformly distributed at certain intervals through peripheral notches of the first shoulder portion 12 between the first inclined surfaces 13, and the specific distribution form can be selected and determined according to practical requirements.

In some embodiments, two first inclined surfaces 13 are formed and are uniformly distributed in a mutually successive manner on the circumference formed by the first shoulder portion 12, so that a total length of the first inclined surfaces 13 is approximately similar to a total circumference of the first shoulder portion 12. Further, in the rotating compensation process of the winding device 100, the length of guide 2 required by the motion along the first inclined surfaces 13 can be met, so that the jumping of guide 2 is avoided and the vibration and noise of the winding device 100 in the cover strip winding process can be avoided.

In some cases, the first inclined surfaces 13 can be matched with the second inclined surfaces 23. Moreover, the length of each of the first inclined surfaces 13 in the peripheral direction is equal to or greater than the length of each of the second inclined surfaces 23 in the peripheral direction. Therefore, in the cover strip winding process of the winding device 100, the first inclined surfaces 13 of drive 1 can remain in contact with the second inclined surfaces 23 of guide 2.

In some embodiments, referring to FIG. 3, drive 1 includes one first shoulder portion 12 which forms a circumference around the driving shaft 11. In addition, the first shoulder portion 12 is provided with two first inclined surfaces 13. The first inclined surfaces 13 are uniformly distributed successively on the circumference of the first shoulder portion 12 around the driving shaft 11. Moreover, an included angle α between a normal vector Fₙ of each of the first inclined surfaces 13 and the rotating direction R of drive 1 is an acute angle.

Through the two first inclined surfaces 13, the length of each of the first inclined surfaces 13 is such that, during the cover strip winding process of the winding device 100, guide 2 is movable relative to drive 1. In this way, the motion compensation of the winding device 100 is maintained when the effective winding diameter of the take-up reel 4 increases. Meanwhile, since the length of each of the first inclined surfaces 13 is longer that a length of the second included surface 23 of guide 2, the second inclined surface 23 remains in contact with the first inclined surface 13 and will not completely separate from the first inclined surface 13. Bouncing of guide 2 caused by the fact that the second inclined surface 23 is not in contact with the first inclined surface 13 of drive 1 is avoided, and the vibration and large noise generated by the winding device 100 in the cover strip winding process are prevented.

Particularly, under the condition of providing the two first inclined surfaces 13 uniformly distributed successively, a rotational stop 14 is formed between adjacent two first inclined surfaces 13 on the first shoulder portion 12. The rotational stop 14 preferably extends on the axial surface parallel to or penetrating through the axial line of the driving shaft 11.

When guide 2 is in contact with the rotational stop 14, the rotational stop 14 forms a first shoulder stop portion for preventing guide 2 from continuing to rotate in the rotating direction R relative to drive 1. At that moment, guide 2 is resting in a stop position of drive 1, and is rotatable together with drive 1 under the driving of drive 1.

Further, when guide 2 and drive 1 rotate together, a clamping manner is as follows: according to the preload applied by the elastic body 3 to guide 2, guide 2 receives force along the first inclined surfaces 13 towards the bottom of drive 1, and forms friction force, between drive 1 and the inclined surface of guide 2, for preventing guide 2 from moving relative to drive 1, so that a motion trend of guide 2 to rotate relative to drive 1 in the rotating direction R is formed. At this moment, since guide 2 is in contact with the rotational stop 14, the rotational stop 14 will achieve a stopping effect of preventing guide 2 from moving relative to drive 1. That is, the rotational stop 14 forms the first shoulder stop portion. The first shoulder stop portion can cooperate with guide 2 to prevent guide 2 from further rotating relative to drive in a direction the same as the rotating direction R.

In a cover strip winding process of the winding device 100, under the tension of the cover strip, guide 2 can further move along the first inclined surface 13, so that guide 2 rotates relative to drive 1 in a direction opposite to the rotating direction R of drive 1.

Referring to FIG. 2 and FIG. 4, guide 2 is provided with a receiving space 21 disposed around the driving shaft 11 and configured to receive the elastic body 3. The receiving space 21 is formed into a first tubular structure with the bottom. In addition, a through hole 26 with the diameter greater than the diameter of the driving shaft 11 is formed in a bottom of the receiving space 21, so that guide 2 can be fitted over the driving shaft 11 through the through hole 26 by inserting the driving shaft 11 into the through hole 26. At the same time, an inner diameter of the receiving space 21 is greater than the outer diameter of the elastic body 3, and the diameter of the through hole 26 in the bottom of the receiving space 21 is smaller than the outer diameter of the elastic body 3, so that the elastic body 3 can be received in the receiving space 21, and thus the elastic body 3 can conveniently apply the axial preload to guide 2.

Under the action of the elastic body 3, for example, under compression, the second inclined surface 23 is in contact with the first inclined surface 13. At the same time, guide 2 is in contact with or attached to the rotational stop 14 of drive 1 and is clamped on the first shoulder portion 12. In this way, guide 2 is installed at the top of drive 1, as seen in FIG. 2. In some embodiments, when the elastic body 3 is a compressed spring, the outer diameter of the elastic body is 1.5 times of the diameter of the through hole 26.

A notch 25 is formed in the outer periphery of the receiving space 21. The notch 25 extends in the radial direction and penetrates through the side wall of the receiving space 21 in the axial direction. Guide 2 is connected with the take-up reel 4 in a relatively nonrotatable manner through notch 25. The quantity of the notches 25 can be selected according to practical requirements. In some embodiments, the quantity of the notches 25 is two. In some cases, a plurality of notches 25 (such as 3, 4, or more) are uniformly distributed in the peripheral direction on the outer periphery of the receiving space 21.

A second shoulder portion 22 is formed at bottom side of the receiving space 21. The quantity and positions of inclined surfaces of the second shoulder portions 22 are in one-to-one correspondence to those of the first inclined surfaces 13. The second inclined surface 23 forms the bottom end surface of the second shoulder portion 22, with a complementary shape matched with the first inclined surfaces 13. That is, the quantity, the plane shape, and the inclined angle (helix angle) of the second inclined surface 23 are the same as those of the first inclined surface 13. For example, when guide 2 is provided with only one second shoulder portion 22, the second inclined surface 23 is formed over the entire circumference of the second shoulder portion 22 around the driving shaft.

When guide 2 is provided with a plurality of second shoulder portions 22, the plurality of shoulder portions 22 form a circumference around the driving shaft 11 and are uniformly distributed on the circumference. The plurality of second shoulder portions 22 can be uniformly distributed successively on the formed circumference, and can also be uniformly distributed at certain intervals through the peripheral notches 241 of the second shoulder portions 22. The specific distribution form can be selected and determined according to practical requirements. When a plurality of second shoulder portions 22 are formed, an arc length of each of the second shoulder portions 22 on the circumference can be shorter than the arc length of the first inclined surface 13.

In some embodiment, referring to FIG. 4, guide 2 is provided with two second shoulder portions 22, and each of the second shoulder portions 22 is provided with one second inclined surface 23. The two second shoulder portions 22 form a circumference around the center axial line of the receiving space 21 and are uniformly distributed on the circumference at a certain interval through peripheral notches 241 therebetween. The second inclined surfaces 23 are formed at the bottom surfaces of the second shoulder portions 22, and the quantity of the second inclined surfaces is correspondingly two in the shown embodiment. In an initial installation state of the winding device 100, guide 2 is installed at the top of drive 1 through the contact between the second inclined surface 23 thereof and the first inclined surface 13 of drive 1.

A vertical surface 24 capable of being parallel to the rotational stop 14 in the initial installation state is formed between the adjacent second inclined surfaces 23, and the vertical surface 24 and the rotational stop 14 are matched in shape. In the initial installation state, the vertical surface 24 is in contact with rotational stop 14, so that a second shoulder stop portion for limiting guide 2 from rotating relative to drive 1 in the rotating direction R is formed. In a process that guide 2 rotates along with drive 1 through contact of the first shoulder stop portion and the second shoulder stop portion, under the action of the elastic body 3, guide 2 is rotatable together with drive 1 under the driving force transferred through drive 1.

Referring to FIG. 5 and FIG. 6, the take-up reel 4 is constructed as a second tubular structure with the top, a through hole 41 formed in the top of the second tubular structure, the through hole 41 having a diameter that is greater than the shaft diameter of the distal end of the driving shaft 11, so that the take-up reel 4 can be positioned over the driving shaft 11 through the through hole 41 for further installing the take-up reel 4 onto the driving shaft 11. A plurality of radially extending protrusions 47 are formed on the inner surface of the tube wall of the take-up reel 4, and the plurality of protrusions 47 are distributed in the peripheral direction on the inner surface of the take-up reel 4, preferably in a uniform distribution manner. In some embodiments, the positions and quantity of the plurality of protrusions 47 of the take-up reel 4 are in one-to-one correspondence to those of the plurality of notches 25 of guide 2.

Alternatively, the positions of the protrusions 47 and corresponding notches 25 are interchangeable. That is, the notches 25 can be disposed on the inner surface of the take-up reel 4, and the protrusions 47 can be disposed on the outer periphery of guide 2.

In the peripheral direction of the inner surface of the take-up reel 4, the size of the protruding portion 47 equals to the size of the notch 25, so that the protruding portion 47 can be inserted into the notch 25. Therefore, the take-up reel 4 can be fitted over the radial outer side of guide 2 through the engagement of the protruding portion 47 and the notch 25 of guide 2, and forms relatively nonrotatable connection with respect to guide 2 and take-up reel 4. Since the protrusions 47 at the inner side of the take-up reel 4 and the notches 25 at the outer side of guide 2 are mutually engaged, and at the same time, the axial motion of the take-up reel 4 is limited, the take-up reel 4 can only do rotating motion. When guide 2 moves along the first inclined surface 13 through the second inclined surfaces 23 thereof, guide 2 can further do axial motion along the protrusions 47 through the notches 25 formed on guide, so that the take-up reel 4 is driven to only do rotating motion.

Referring to FIG. 5, an axially extending cut-out portion 43 is further disposed on the outer periphery of the take-up reel 4. A cover strip receiving fin 44 is vertically disposed on the outermost circumference of the cut-out portion 43. The cover strip forms loop-shaped collar at its initial position, the collar is matched with the fin 44 in shape, and the cover strip is fixed onto the take-up reel 4 by fitting the collar over the cover strip receiving fin 44.

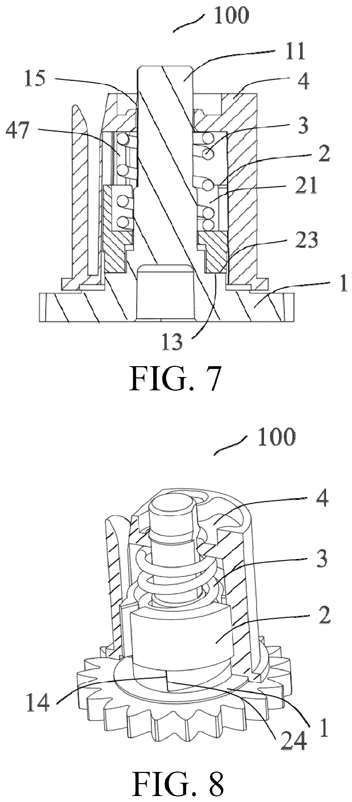

FIG. 7 is a sectional view of the winding device 100 in an assembly state. Referring to FIG. 7, after the assembly of the winding device 100 is completed, guide 2, the elastic body 3 and the take-up reel 4 are sequentially installed or fitted over the driving shaft 11 of drive 1.

The top of the take-up reel 4 abuts against the bottom surface of the protruding rib portion 15 of the driving shaft 11 near the take-up reel 4, so that the protruding rib portion 15 prevents axial motion of the different elements of the winding device 100. The elastic body 3, for example, a compressed spring, is disposed in the receiving space 21 of guide 2, and is positioned between the take-up reel 4 and guide 2, so that a preload in an axial direction towards the driving base plate 110 is applied onto guide 2 to axially press guide 2. Due to the preload of the elastic body 3, the second inclined surface 23 is attached to the first inclined surface 13. At the same time, the protrusion 47 on the inner surface of the take-up reel 4 is inserted into the notch 25 in a corresponding position of guide 2, and the take-up reel 4 and guide 2 are thus rotationally locked so that the take-up reel 4 is rotatable along with guide 2 under the driving of drive 1.

In an initial state, the cover strip is wound onto the take-up reel 4, and a feeding component feeds the cover strip at a constant angle each time. When the tension received by the wound cover strip does not exceed a preset threshold, referring to FIG. 8, under the axial extrusion of the elastic body 3, the vertical surface 24 of guide 2 and the rotational stop 14 are in contact with each other. At this moment, the take-up reel 4 is in a normal working state. That is, the take-up reel 4 rotates together with drive 1.

After the take-up reel 4 winds the cover strip for a circle, the effective winding diameter of the take-up reel 4 correspondingly increases, the linear speed of the take-up reel 4 increases correspondingly, and the length increases of the wound cover strip with each rotation of the take-up reel 4. At this moment, the length of the wound cover strip is increased, and the length of the cover strip fed as each rotation of the feeding component is constant, so the tension of the cover strip between the winding device 100 and the administration device is increased.

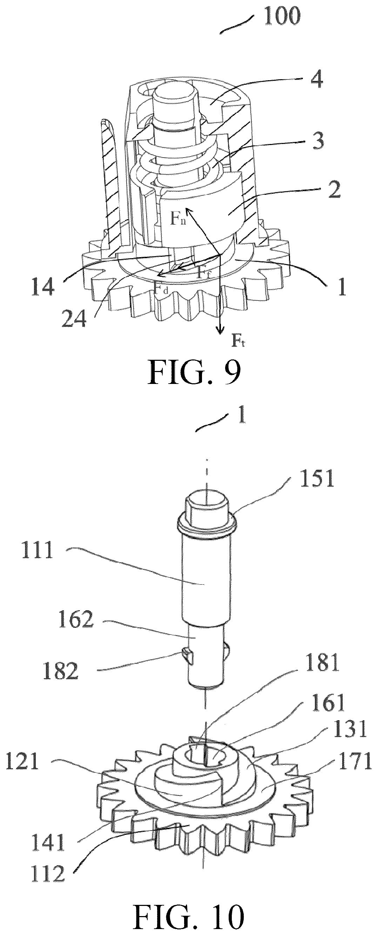

When the tension increases to a preset value, based on the tension received by the cover strip, referring to FIG. 9, guide 2 can overcome friction force F_{f} generated due to the preload Fₜ of the elastic body 3 between guide 2 and the first inclined surface 13 and component force F_{d} of the preload Fₜ along the first inclined surface towards the bottom of drive 1, so that guide and the take-up reel 4 rotate together relative to drive 1 in a direction opposite to the rotating direction R, and at the same time, guide 2 upwards moves along the first inclined surface 13. The friction force F_{f} equals to a product of a friction factor between the first inclined surface 13 and the second inclined surface 23 and a component of the preload Fₜ on a normal vector of the first inclined surface 13.

In operation, the winding device 100 is in a motion compensation working state. Specifically, the take-up reel 4 connected with guide 2 in a relatively nonrotatable manner rotates relative to drive 1 in a direction opposite to the rotating direction R of drive 1 under the driving of guide 2. That is, the take-up reel 4 will rotate reversely relative to drive 1 due to the tension, so that the motion compensation for the take-up reel 4 is realized. However, at this moment, the take-up reel 4 still winds the cover strip. That is, at this moment, the rotating motion of the take-up reel 4 and guide 2 is still rotation in the rotating direction R of drive 1. Therefore, based on the tension received by the cover strip, the winding device 100 can overcome the friction force between the first shoulder portion 12 and guide 2 and the component generated by the preload along the first inclined surface 13, so that the second inclined surface 23 of guide 2 and the first inclined surface 13 of drive 1 can move relative to each other, thereby realizing motion compensation for the take-up reel 4.

In another embodiment, winding device 100 comprises a driver 1 where the driving shaft and the driving base plate are separately formed, but all of the other components of winding device 100 are as previously described. Specifically, referring to FIG. 10, the separately formed driver 1 comprises a driving shaft 111, a first shoulder portion 121 and a step portion 171. The driving shaft 111 detachably installed in the center of the driving base plate 112 of drive 1.

Referring to FIG. 10 to FIG. 13, drive 1 includes the driving shaft 111 and the driving base plate 112. A through hole 161 is formed in the center of the driving base plate 112. A plurality of radially extending and axially penetrating grooves 181 are formed in the inner surface at the position of the through hole 161. The grooves 181 are uniformly distributed in the peripheral direction in the inner surface of the through hole 161. In some cases, two grooves 181 are formed. At the same time, the driving shaft 111 is constructed as a stepped shaft, where a diameter of a shaft 162 of the driving shaft 111 at the end near the driving base plate 112 is smaller than the diameter of the through hole 161, so that the shaft 162 can be inserted into the through hole 161. In addition, the end of the shaft 162 near the driving base plate 112 is provided with a plurality of uniformly distributed wedges 182, and all of the wedges 182 are uniformly distributed along the outer periphery of the shaft 162. In some instances, the positions and quantity of the wedges 182 are in one-to-one correspondence to those of the installing grooves 181. In addition, in the peripheral direction, the size of the wedges 182 is smaller than the size of grooves 181, so that each wedge 182 can pass through the groove 181 in the corresponding position, and the wedges 182 can be inserted into the through hole 161 of drive 1 together with the shaft 162.

At the same time, a hole 183 with a diameter greater than that of the through hole 161 is formed in the lower surface of the center of the bottom of drive 1. The hole 183, the through hole 161 and the groove 181 jointly form an arc-shaped bump 184 positioned inside the driving base plate 112. One or more wedge receiving notches 185 capable of being engaged with wedge 182 is formed in the bottom surface of the bump 184. An angle is formed by the center line of the wedge receiving notch 185 and the center axis of the installing groove 181 in a space. In some instances, the angle is 90°.

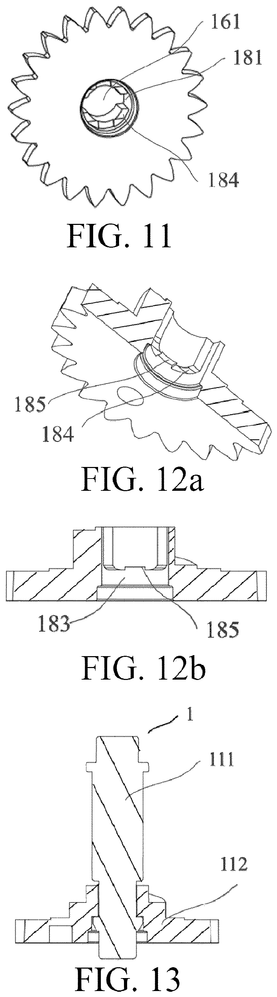

Specifically, a process of fixing the driving shaft 111 to the driving base plate 112 includes that after the shaft 162 and the wedge 182 are inserted into the through hole 161 of the driving base plate 112, the driving shaft 111 is rotated by a certain angle, so that the wedge 182 reaches the corresponding position of the wedge receiving notch 185 in the hole 183. Then the driving shaft 111 is lifted in the axial direction for pressing the wedge 182 into the wedge receiving notch 185, allowing the wedge 182 to be connected and fixed into the wedge receiving notch 185, as shown in FIG. 13. In this way, the installed and fixed driving shaft 111 does synchronous rotating motion along with drive 1. In addition, a protruding rib portion 151 on the driving shaft 111 is constructed to present a circular ring shape with a flat lower surface. In some embodiments, the surface of the top of the take-up reel 4 is in planar contact with the protruding rib portion 151, so that friction between a top surface of the take-up reel 4 and the protruding rib portion 151 is plane friction, thereby reducing and distributing the friction force between the take-up reel 4 and the driving shaft 111.

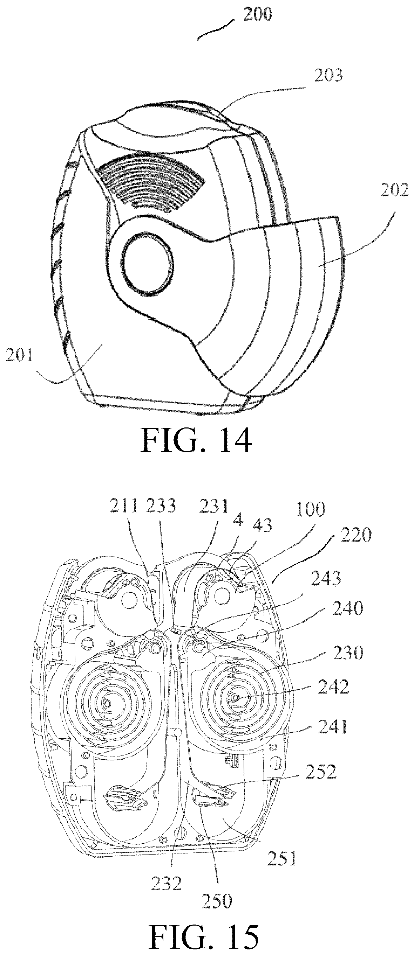

According to another aspect of the disclosure, a medicine dispenser 200 as shown in FIG. 14 is also provided. Referring to FIG. 14 to FIG. 16, the medicine dispenser 200 includes a housing 201, a cover body 202, a suction nozzle 203, and an intake manifold 210 and a medicine dispensing device 220 which are disposed inside the housing. The intake manifold 210 is installed on a specific position of the medicine dispensing device 220, and the cover body 202 is mechanically coupled with the medicine dispensing device 220. A user can trigger the medicine dispensing device 220 inside the housing to move by opening the cover body 202. When the cover body 202 is completely opened, the medicine dispensing device 220 can dispense the medicine, such as powdered medicine into the corresponding position of the airway of the intake manifold 210 in the normal working state, so that the user can inhale the medicine powder through the suction nozzle 203 via the intake manifold 210.

Illustratively, the cover body 202 is mechanically coupled with the medicine dispensing device 220 through a transmission mechanism (not shown). Driving power can be input into the medicine dispensing device 220 through the rotation of the cover body 202. In addition, the intake manifold 210 is installed on a manifold installing portion 211 in a specific position of the medicine dispensing device 220, so that in the normal working state, the medicine strip is dispensed by the medicine dispensing device 220 for administration, the medicine carried on the medicine strip and exposed in the air can face the airway of the intake manifold 210.

In the descriptions below, the specific structure of the medicine dispensing device 220 and the medicine dispensing process will be described in combination with the medicine strip 230. The medicine strip 230 includes a cover strip 231, a base carrier strip 232 and a medicine receiving space 233 which is positioned between the cover strip and the base carrier strip and is able to accommodate the powdered medicine. In addition, the base carrier strip 232 and the cover strip 231 at the end portion of the medicine strip 230 in its length direction are in a state of being separated from each other.

Referring to FIG. 15 to FIG. 16, the medicine dispensing device 220 includes an administration device 240, a stripping device (not shown), a base carrier strip take-up device 250 and a winding device 100. The winding device 100 is constructed to wind the cover strip 231 in the medicine dispensing device 220.

The administration device 240 is constructed to dispense the medicine. Specifically, the administration device 240 includes a medicine strip receiving cavity 241 and a medicine strip installing shaft 242. The medicine strip 230 can be received in the medicine strip receiving cavity 241 after being wound, and is simultaneously installed and fixed onto the medicine strip installing shaft 242, so that the medicine strip can move around the medicine strip installing shaft 242 under the action of external force or the action of tensile force of the winding device 100, so as to realize the motion of the medicine strip 230 in the housing and the medicine dispensing action. The stripping device is constructed to strip the medicine strip 230 near the airway.

The base carrier strip take-up device 250 is constructed to wind up the stripped base carrier strip 232. The base carrier strip take-up device 250 includes a base carrier strip receiving cavity 251 and a winding shaft 252. The end portion of the base carrier strip 232 is fixed onto the installing portion of the winding shaft 252, and the stripped base carrier strip 232 can be wound up onto the winding shaft 252 under the rotating of the winding shaft 252 and thus be received into the base carrier strip receiving cavity 251.

The winding device 100 is constructed to wind the stripped cover strip 232. A specific structure and the working process of the winding device 100 are as previously described herein. Referring to FIG. 15, an end portion of the cover strip 232 in a separated state is fixed onto the cover strip fin 43 of the take-up reel 4 of the winding device 100, and is wound onto the outer surface of the take-up reel 4 through the action of the take-up reel 4.

In addition, in a process of winding the stripped cover strip 232, the take-up reel 4 is rotatable relative to drive 1 to realize the motion compensation, so that the length consistency of the wound cover strip 232 as each rotation of the take-up reel 4 is ensured, thereby ensuring that the relative positions of the medicine receiving cavity and the intake manifold 210 remains unchanged. Therefore, the medicine dispenser 200 can realize the medicine dispensing through the rotation of the cover body 202, the administration device 240, the winding device 100 and the base carrier strip winding device 250, and can realize the medicine availability through the mutual effects of the above components and devices.

When the cover body 202 rotates, the medicine dispensing device 220 is triggered to start the medicine dispensing work. When the medicine dispensing device 220 is triggered, referring to FIG. 15, an index wheel 243 of the administration device 240 starts to rotate, so that the medicine strip 230 is driven to rotate around the medicine strip installing shaft 242, and the medicine strip 230 which does not rotate to the medicine strip installing shaft 242 moves in the housing in a direction towards the airway of the intake manifold 210. At the same time, the winding device 100 will synchronously rotate along with the administration device 240, the base carrier strip take-up device 250 also synchronously rotates. When the cover strip 231 and the base carrier strip 232 of the wound medicine strip 230 are stripped and separated through the index wheel 243 of the administration device 240 and the stripping device, the cover strip 231 is wound onto the take-up reel 4 of the winding device 100 once it is stripped off under the action of tensile force formed by the rotation of the take-up reel 4 of the winding device 100.

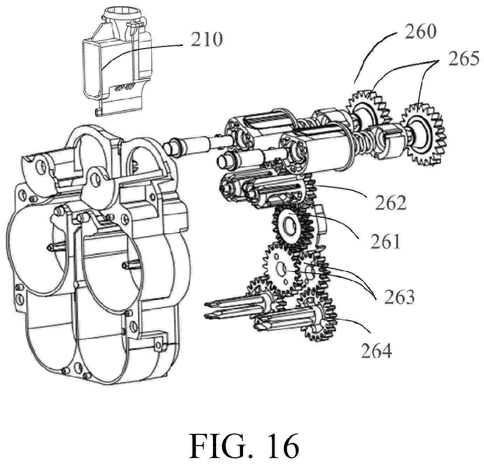

At the same time, the base carrier strip 232 separated through the stripping effect is wound onto the winding shaft 252 of the base carrier strip winding device 250. At this moment, the medicine received in the medicine receiving cavity 233 is inhaled by a patient through the airway of the intake manifold 210 and the suction nozzle 203 through an opening formed after the stripping. In general, the medicine dispensing and the medicine availability are realized through the synchronous cooperative rotation of the administration device 240, the winding device 100 and the base carrier strip winding device 250. A gear transmission system 260 that enables the synchronous cooperative rotation among the administration device 240, the winding device 100 and the base carrier strip device 240 will be described in detail hereafter in combination with FIG. 16.

Referring to FIG. 16, the gear transmission system 260 includes a triggering gear 261, an administration gear 262, a first transmission gear 263, a driving gear 264 and a base carrier strip winding gear 265. The driving gear 264 can be a gear for forming the driving base plate 110 or the driving base plate 112 as shown in FIG. 3 or FIG. 10, respectively. That is, the driving base plate 110 of drive 1 as shown in FIG. 3 is constructed as the gear or the driving base plate 112 of drive 1 as shown in FIG. 10 is constructed as the gear. Specifically, a power transmission process of the gear transmission system 260 will be described in detail hereafter.

The gear transmission system 260 is mechanically coupled with the cover body 202 through the triggering gear 261 via the transmission mechanism (not shown), and can receive the input driving power under the rotating triggering of the cover body 202. The administration gear 262 is engaged with the triggering gear 261, and can rotate simultaneously under the driving of the triggering gear 261 when the triggering gear 261 rotates. In addition, the index wheel 243 in the administration device 240 is fixed to the administration gear 262, and the index wheel 243 rotates along with the administration gear 262, so that the index wheel 243 rotates synchronously under the driving of the triggering gear 261 for driving the medicine strip 230 to dispense the medicine.

At the same time, the base carrier strip winding gear 264 is engaged with the first transmission gear 263, and the first transmission gear 163 is engaged with the triggering gear 261. That is, the base carrier strip winding gear 264 is matched with the triggering gear 261 via the first transmission gear 263, so that the base carrier strip winding gear 264 can do synchronous rotation with the triggering gear 261 via the first transmission gear 163. At the same time, the base carrier strip winding shaft 251 is disposed on the base carrier strip winding gear 264, so that the base carrier strip winding shaft 251 can rotate along with the base carrier strip winding gear 264 to wind the stripped base carrier strip 232, thereby realizing the synchronous rotation of the base carrier strip winding device 250 and the administration device 240.

The driving gear 265 is engaged with the administration gear 262, and can realize the synchronous rotation under the driving of the administration gear 262. Therefore, rotating motion is input to the winding device 100, and the winding device 100 winds the stripped cover strip 231 under the driving of the driving gear 265. When the effective winding diameter of the take-up reel 4 increases, as described in each embodiment according to the first aspect of the disclosure, the take-up reel 4 is rotatable relative to the driving gear 265 to compensate the possible length of the cover strip 231 that can be additionally wound by the take-up reel 4 as each rotation of the driving gear 265 due to the increase of the outermost circle diameter, and the length consistency of the cover strip 231 wound by the take-up reel 4 as each rotation of the driving gear 265 is ensured, thereby avoiding the change of the practical position of the medicine exposed to the air due to the tensile force of the take-up reel 4, and further avoiding the problem that a user cannot acquire the medicine due to misalignment of the medicine and the airway position of the intake manifold 210. At the same time, the length of the cover strip 231 wound as each rotation of the take-up reel 4 is consistent.

Therefore, the advancing tension provided for the medicine strip 230 by the take-up reel 4 or the tension of the medicine strip 230 between the take-up reel 4 and the administration device 240 is basically constant, thereby effectively avoiding the fracture of the medicine strip 230 due to excessive tension.

The disclosure is not limited to above foregoing implementations, and can be properly modified without departing from the scope of the disclosure. For example, in the above implementations, it has been described that the second inclined surface is disposed on the take-up reel, and guide can also be integrally formed with the take-up reel. It can be obviously seen from the above disclosure that the solutions of the disclosure can be modified in various manners. These modifications should not be considered as a departure from the spirits and scope of the disclosure, and all these modifications that can be made by a person skilled in the art are intended to fall within the scope of the appended claims.

## Claims

1. A winding device for a cover strip, comprising:
a driver comprising a driving shaft around which a first shoulder portion having a first inclined surface is disposed, configured to be rotatable in a rotating direction;
a guide installed on the driving shaft, and having a second inclined surface matched with the first inclined surface of the first shoulder portion;
a take-up reel connected with the guide in a relatively nonrotatable manner; and
an elastic body received between the guide and the take-up reel for applying a preload to the guide,
wherein the winding device is constructed such that the take-up reel is rotatable in the rotating direction under the driving of the driver, when the force acting on the take-up reel via the cover strip is greater than a preset value, the take-up reel is also rotatable relative to the driver.

2. The winding device of claim 1, wherein an included angle between a normal vector of the first inclined surface and the rotating direction of the driver is an acute angle.

3. The winding device of claim 2, wherein one first inclined surface is disposed on the driver, and the first inclined surface is distributed over the entire circumference of the first shoulder portion around the driving shaft.

4. The winding device of claim 2, wherein a plurality of first inclined surfaces is disposed on the driver, and each of the first inclined surfaces is uniformly distributed in the peripheral direction over the entire circumference of the first shoulder portion around the driving shaft.

5. The winding device of claim 4, wherein two first inclined surfaces are disposed on the driver.

6. The winding device of claim 5, wherein the elastic body is a compressed spring.

7. The winding device of claim 6, wherein guide comprises a receiving space disposed around the driving shaft and into which the compressed spring is positioned.

8. The winding device of claim 7, wherein the receiving space is formed as a first tubular recess having a bottom surface, a through hole is formed in the bottom surface, and the driving shaft passes through the through hole.

9. The winding device of claim 8, wherein an axially penetrating notch is formed on an outer periphery of the receiving space.

10. The winding device of claim 9, wherein the take-up reel is constructed into a second tubular structure with a top surface, a through hole is formed in the top surface, a protruding portion is formed on an inner surface of the take-up reel, and the take-up reel is fitted over the outer periphery of the guide through the engagement of the protruding portion and the notch.

11. The winding device of claim 1, wherein a protruding rib portion is disposed at an end of the driving shaft distal to the base, and a top surface of the take-up reel is abutted against a bottom surface of the protruding rib portion.

12. The winding device of claim 11, wherein the take-up reel is axially provided with a cut-out portion, and a cover strip fin is vertically disposed on the outermost circumference of the cut-out portion.

13. The winding device of any one of claims 1 to 12, wherein the driver comprises a driving base plate positioned at the base thereof, and the driving shaft and the driving base plate are integrally formed or separately formed.

14. The winding device of claim 13, wherein in a case that the driving shaft and the driving base plate are separately formed, the driving shaft is detachably installed on the driving base plate.

15. The winding device of claim 13, wherein the driving base plate is constructed as a gear.

16. The winding device of claim 14, wherein a second shoulder portion is formed at the bottom of the guide, and the second inclined surface is formed on the second shoulder portion.

17. The winding device of claim 16, wherein a position of the second shoulder portion is in one-to-one correspondence to the first inclined surface.

18. The winding device of claim 16, wherein a first shoulder stop portion and a second shoulder stop portion that are mutually clamped are respectively formed on the first shoulder portion and the second shoulder portion.

19. A medicine dispenser, comprising the winding device of any one of claims 1 to 18.
